# NEUE EUROPÄISCHE PATENTSCHRIFT

(11) **EP 0 859 585 B2**
(45) Veröffentlichungstag und Bekanntmachung des Hinweises auf die Entscheidung über den Einspruch: **14.06.2006**
(45) Hinweis auf die Patenterteilung: 11.09.2002
(21) Anmeldenummer: 96938121.9
(22) Anmeldetag: 06.11.1996
(51) Int. Cl.: A61Q 5/00

(54) **HAARBEHANDLUNGSMITTEL**
HAIR TREATMENT COMPOSITION
COMPOSITIONS DE TRAITEMENT CAPILLAIRE

(30) Priorität: 06.11.1995 DE 19541329
(43) Veröffentlichungstag der Anmeldung: 26.08.1998
(73) Patentinhaber: BASF Aktiengesellschaft, 67056 Ludwigshafen (DE)
(72) Erfinder: NGUYEN KIM, Son, D-69502 Hemsbach (DE); SPERLING, Karin, D-67433 Neustadt (DE)
(74) Vertreter: Kinzebach, Werner
(86) Internationale Anmeldenummer: PCT/EP1996/004857
(87) Internationale Veröffentlichungsnummer: WO 1997/017052

(56) Entgegenhaltungen:
- EP-A- 0 195 895
- EP-A- 0 282 720
- EP-A- 0 760 235
- WO-A-93/25179
- WO-A-95/23579
- CH-A- 535 579
- DE-A- 3 140 134
- DE-A- 4 304 697
- DE-A- 4 408 727
- GB-A- 2 188 948

## Beschreibung

Die vorliegende Erfindung betrifft Haarbehandlungsmittel und insbesondere Haarfestigungsmittel in Form eines Haarsprays.

In Wasser lösliche oder in Wasser dispergierbare Polymere, beispielsweise Polyester, Polyamide oder Polyurethane, gewinnen aufgrund ihrer besonders niedrigen Viskosität in Wasser bzw. in Wasser/Ethanol immer mehr an Bedeutung. So sind wasserlösliche Polyurethane, die Carboxylgruppen aufweisende Diole einpolymerisiert enthalten, aus der US-A-3,412,054 und 3,658,939 bekannt.

Sie werden als Klebstoff, Beschichtungsmittel und in Drucktinten verwendet. Sulfonat- und/oder Carboxylatgruppen enthaltende Polyurethane, die in Wasser dlspergierbarsind, sind aus der DE-A-15 70 615 bekannt. Sie werden beispielsweise zur Beschichtung und zum Imprägnieren von Textillen, Leder, Papier, Holz und Metallen verwendet. Aus den Schutzrechten US-A-4,300,580, US-A-3,734,874, DE-A-26 33 418 und WO-A-89/07118 sind NaSO₃-Gruppen enthaltende Polyester bekannt, deren Hauptkette durch Kondensationsreaktion aufgebaut ist und die durch Hydrolyse der Estergruppierungen zu kürzeren Segmenten abgebaut werden können.

Weiter ist bekannt, daß Maleinsäureanhydrid und Trimellitsäureanhydrid zur Herstellung von was serlöslichen Estern verwendet werden können. Die Anhydridgruppierung stellt Carboxylgruppen zur Verfügung, welche durch Neutralisation mit Aminen, Metallhydroxiden und Metallcarbonaten in Carboxylatgruppen überführt werden, wodurch Wasserlöslichkeit erzielt wird. Aus der DE-A-26 37 167 und der US-A-3,523,998 ist bekannt, daß auch Polycarbonsäuren und ihre Anhydride als Polymerisatkomponenten dazu beitragen können, Polyester wasserlöslich zu machen. Eine kosmetische Anwendung derartiger Polymere ist bisher jedoch noch nicht beschrieben.

In der Kosmetik werden Filmbildnerpolymere zur Festigung, Strukturverbesserung und Formgebung der Haare verwendet. Die Haarbehandlungsmittel enthalten im allgemeinen eine Lösung des Filmbildners in einem Alkohol oder einem Gemisch aus Alkohol und Wasser.

Die US-A-4,743,673 beschreibt hydrophile Polyurethanpolymere mit Carboxygruppen im Polymerrückgrat. Diese Polyurethane sind aufgebaut aus einer Polyolkomponente,bei der es sich um ein Alkylenglycol, ein Polyoxyalkylenglycol oder ein lineares Polyesterdiol handeln kann, einer Carbonsäureesterkomponente mit Hydroxy- oder Aminogruppen und einem organischen Isocyanat oder lsocyanat-Vorläüfer. Das Polyurethan enthält also an das Polymerrückgrat gebundene Estergruppen, die anschließend durch 30- bis 60-minütiges Erhitzen mit einer starken Base, wie Natrium- oder Kaliumhydroxid, unter Rückfluß verseift werden. Das erhaltene Produkt ist sowohl in Wasser als auch in Ethanol nicht mehr klar löslich. Dieser ist nicht mehr in Wasser, sondern nur noch in niedrigen aliphatischen Alkoholen und anderen Lösungsmitteln löslich. Insbesondere bei Verwendung eines Polyesterdiols als Polyolkomponente erfolgt aufgrund der Behandlung mit der starken Base unter Rückflußbedingungen nicht nur eine Verseifung der Estergruppen der Carbonsäureesterkomponente, sondern auch eine Verseifung der in der Polyurethankette enthaltenen Estergruppierungen. Es kommt somit zur Spaltung der Polyurethankette und zu einer drastischen Verringerung des Molekulargewichts der Polyurethane. Eine Anwendung der Polyurethane in Haarsprays ist zwar erwähnt. In der Praxis sind die mit diesen Polyurethanen erhaltenen Filme für die

Haarkosmetik aber nicht brauchbar, well sie entweder wasserunlöslich sind oder ein zu geringes Molekulargewicht und somit unzureichende Festigungswirkung besitzen.

Die DE-A-42 25 045 beschreibt die Verwendung von wasserlöslichen oder In Wasser dispergierbaren, anlonischen Polyurethanen als Haarfestiger. Diese Polyurethane sind aufgebaut aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat.

Sie besitzen eine Glastemperatur von mindestens 15°C und Säurezahlen von 12 bis 150. Als Komponente a) kommen vorzugsweise Polyethylenglycol, Neopentylglycol und Polyesterole zur Anwendung. Bevorzugte Komponenten (b) sind Dimethylolpropansäure, ein Kondensat aus Pyromellithsäuredianhydrid und Neopentylglykol und ein Kondensat aus 5-Natriumsulfonatoisophthalsäure mit Neopentylglycol.

Die DE-A-42 41 118 beschreibt die Verwendung von kationischen Polyurethanen und Polyharnstoffen als Hilfsmittel in kosmetischen und pharmazeutischen Zubereitungen. Siefinden insbesondere Anwendung als Filmbildner in Haarfestigern. Sie sind aufgebaut aus
a) mindestens einem Diisocyanat, welches bereits vorher mit einer oder mehreren Verbindungen, die zwei oder mehrere aktive Wasserstoffatome pro Molekül enthalten, umgesetzt sein kann und
b) mindestens einem Diol, primären oder sekundären Aminoalkohol, primären oder sekundären Diamin oder primären oder sekundären Triamin mit einem oder mehreren tertiären, quartären oder protonierten tertiären Aminstickstoffatomen.

Die Polymere besitzen eine Glasübergangstemperatur von mindestens 25°C und eine Aminzahl von 50 bis 200, bezogen auf die nicht-quaternisierten oder protonierten Verbindungen.

Die EP-A-619 111 beschreibt die Verwendung von Polyurethanen mit Carboxylatgruppen in Haarfixierungsmitteln. Als Verbindung, welche die Carboxylatgruppen zur Verfügung stellt, enthalten diese Polyurethane eine Verbindung der Formel 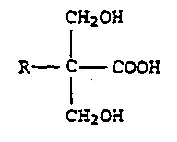 worin R für ein Wasserstoffatom oder eine C₁-C₂₀-Alkylgruppe steht. Zumindest ein Teil der Carbonsäuregruppen wird dabei mit einer organischen oder anorganischen Base neutralisiert, um die Anzahl an Carboxylatgruppen zur Verfügung zu stellen, die erforderlich ist, um das Polyurethan in Wasser oder einem Gemisch aus Wasser und einem polaren organischen Lösungsmittel löslich zu machen.

Die EP-A-636 361 beschreibt kosmetische Mittel, welche als Filmbildner ein Polymer mit mindestens einer Polysiloxanelnhelt und mindestens einer Polyurethan-und/oder Polyharnstoffeinheit mit anionischen oder kationischen Gruppen umfaßt. Aufgrund der Siloxaneinheit sind die Filme im Vergleich zu nicht-siloxanhaltigen Polymeren glatter und liefern besseren Griff und die Haare werden geschmeidiger. Analog zu den erwähnten Polyurethanen, ist die Auswaschbarkeit dieser siloxanhaftigen Filmbildner ebenfalls nicht zufriedenstellend, wenn die Polymere einen K-Wert > 24 aufweisen. Wenn der K-Wert dagegen kleiner als 24 ist, ist die Auswaschbarkeit akzeptabel, die Festigungswirkung aber ungenügend.

Weiterhin ist bekannt, daß bei der Formulierung von Haarbehandlungsmittel, z.B. Haarspray, eine SiliconVerbindung (häufig Polydimethylsiloxan; CTFA-Bezeichnung: Dimethicon, z.B. Abil 10-100000 Fa. Goldschmidt) zugesetzt wird. Infolge des ausgeprägten hydrophoben Charakters der Produkte haben sie positive Auswirkungen auf die Filmeigenschaften des Haarbehandlungsmittels. Aufgrund der hohen Hydrophobie sind die Polysiloxane jedoch in Wasser sowie Ethanol sehr schlecht löslich. Durch Phasen-Separation der Siliconverbindung aus einer Ethanol-Wasser-Lösung beim Trocknen ist der Film trüb und ungleichmäßig verteilt. Die Haare sind daher schlecht auswaschbar und es verbleibt ein unlöslicher Silicon-Rest am Haar, d.h. die Haare sind schwer und "fettig".

Haarfestigungsmittel werden im allgemeinen in Form von wäßrig-alkoholischen Lösungen auf die Haare aufgesprüht. Nach dem Verdampfen des Lösungsmitttels werden die Haare an den gegenseitigen Berührungspunkten vom zurückbleibenden Polymer in der gewünschten Form gehalten. Die Polymere sollen einerseits so hydrophil sein, daß sie aus dem Haar ausgewaschen werden können, andererseits aber sollen sie hydrophob sein, damit die mit den polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht (K-Wert >25) und eine relativ hohe Glastemperatur (mindestens 15°C) besitzen. Polymere, welche diese Forderungen erfüllen, sind aber aufgrund des höheren Molekulargewichtes schlechter auswaschbar.

Bei der Formulierung von Haarfestigem ist außerdem zu berücksichtigen, daß aufgrund der Umweltbestimmungen zur Kontrolle der Emission flüchtiger organischer Verbindungen (VOC = volatile organic compounds) in die Atmosphäre eine Verringerung des Alkoholund Treibmittelgehalts erforderlich ist.

Die In den oben erwähnten Publikationen beschriebenen Polymere erfülien diese einander entgegenstehenden Anforderungen nur teilweise. So besitzen die in der DE-A-42 25 045 und 42 41 118 sowie in der EP-A-619 111 und EP-A-636 361 beschriebenen Polymere aufgrund ihres hohen Molekulargewichtes einerseits die gewünschte Festigerwirkung. Andererseits aber sind sie nur unzureichend auswaschbar. Die in der US 4,743,673 beschriebenen Polymere besitzen aufgrund Ihres durch Verseifen der Estergruppen bewirkten geringen Molekulargewichtes nicht die erforderliche Festigerwirkung.

Der vorliegenden Erfindung liegt daher die Aufgabe zugrunde, Haarbehandlungsmittel zur Verfügung zu stellen, die einerseits als Haarfestiger brauchbar sind, andererseits aber auch verbesserte Auswaschbarkeit (Redispergierbarkeit) besitzen.

Überraschenderweise wurde nun gefunden, daß diese Aufgabe gelöst wird, wenn man die als Haarfestiger brauchbaren Polymere zusammen mit einem in Wasser oder einem Wasser-Alkohol-Gemisch löslichen Salz aus einer organischen Säure und einem Amin zur Anwendung bringt. Auf diese Weise werden sogar gut auswaschbare siloxanhaltige Haarbehandlungsmittel erhalten.

Ein erster Gegenstand der vorliegenden Erfindung ist daher ein Haarbehandlungsmittel gemäß Anspruch 1.

Vorzugsweise kommen für die Bildung des Salzes folgende Säuren zur Anwendung:
- 1,2-, 1,3- oder 1,4-Benzoldicarbonsäure (Phthalsäure, Isophthalsäure oder Terephthalsäure), 1,2,4- oder 1,3,5-Benzoltricarbonsäure,
- 5-Hydroxy-isophthalsäure;
- Benzol-1,3-dicarbonsäure-5-sulfonsäure (5-SO₃H-Isophthalsäure);
Ein weiteren Gegerstand der Erfindung ist ein Haarbehandlungs mittel gemäß Anspruch 2. Als Siloxonhaltige Säuren sind berorzugt:
1. Einwertige, siloxanhaltige Säuren der Formel

   R¹―NHCO―R²―COOH (IV)

   worin R¹ für 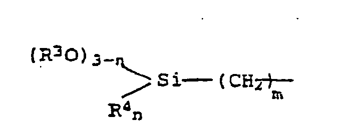 mit n = 0-3
   und m = 1-6 steht, wobei R³ und R⁴ für C₁-C₆-Alkyl stehen und R² für einen der folgenden Reste steht: R² = -(CH₂)ₘ- mit m = 2-6, insbesondere 2 und 3 ,
   ―CH=CH― 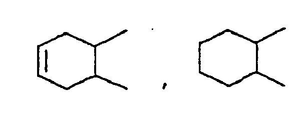 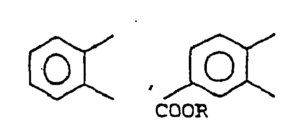 oder der Formel 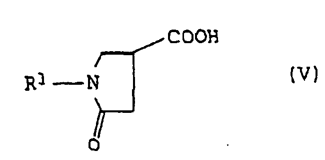 worin R¹ die oben angegebenen Bedeutungen besitzt.
   Beispiele für Verbindungen der Formeln IV und V sind: 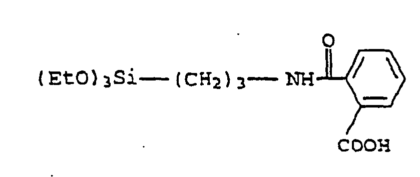 und 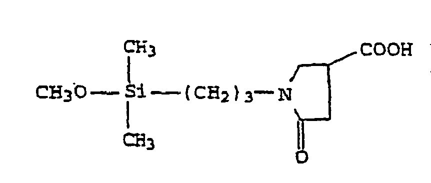
   Die Carbonsäuren der Formel IV sind erhältlich durch Umsetzung eines einwertigen siloxanhaltigen Amins der Formel R¹-NH₂ mit dem entsprechenden Anhydrid 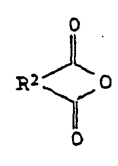 wobei R¹ und R² die obigen Bedeutungen besitzen. Die Verbindungen der Formel V sind erhältlich durch Kondensation eines Amins R¹NH₂ (R¹ wie oben definiert) mit Itaconsäure.
2. Zweiwertige, siloxanhaltige Säuren der Formel: 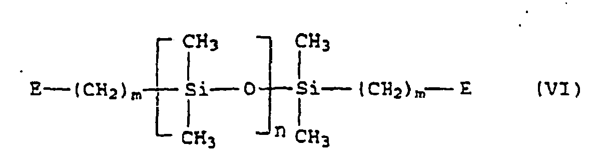 worin E für COOH oder 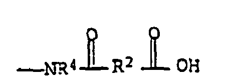 steht, wobei R² die oben angegebenen Bedeutungen besitzt und R⁴ für H oder C₁-C₆-Alkyl steht; m für 1 bis 6 und n für 1 bis 50 steht. Wenn E für COOH steht, steht n vorzugsweise für 1 bis 10 und wenn E für 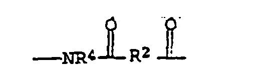 steht, steht n vorzugsweise für 5 bis 35.
   Beispiele für Carbonsäuren der Formel VI sind: 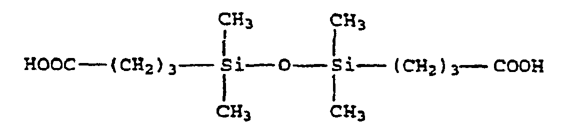 der Fa. Geleet und die Tegomer C-Si-Typen, z.B. C-Si 2142 und C-Si 2342 der Fa. Goldschmidt; oder zweiwertige, siloxanhaltige Säuren der Formel 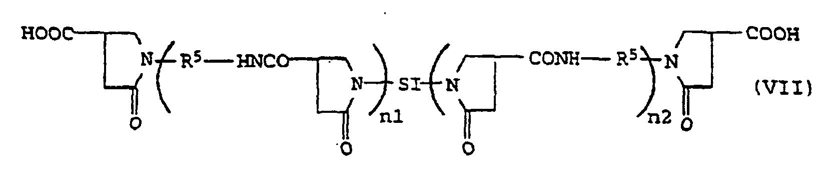 worin R⁵ für C₂-C₄-Alkylen steht und n1 + n2 = 0-20, vorzugsweise = 0 und SI für 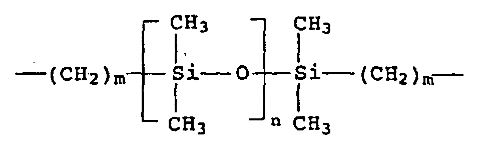 steht, wobei m für 1 bis 6 und n für 1 bis 10 steht.
   Carbonsäuren der Formel VII, worin n1 + n2 = 0, werden durch Kondensation von Itaconsäure mit dem entsprechenden Diamin im Molverhältnis von 2:1 erhalten. Wenn n1 + n2 ≠0, werden zunächst Itaconsäure und siloxanhaltiges Diamin und dann das erhaltene Kondensationsprodukt mit einem siloxanfreien Diamin in den jeweils entsprechenden Molverhältnissen umgesetzt. Wenn z.B. n1 + n2 = 5, werden 7 Mol ltaconsäure mit 1 Moi siloxanhaltigem Diamin und dann mit 5 Mol siloxanfreiem Diamin, z.B. Ethylendiamin, umgesetzt. Die Kondensation mit dem siloxanfrelen Diamin erfolgt im allgemeinen bei 150-170°C.
3. mehrwertige, siloxanhaltige Säuren der Formel: 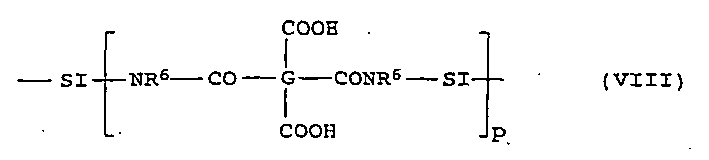 worin
   R⁶ für H oder C₁-C₆-Alkyl steht, SI für die oben angegebenen Bedeutungen steht, p für 1 bis 100 steht und G für den tetravalenten Rest eines Dianhydrids 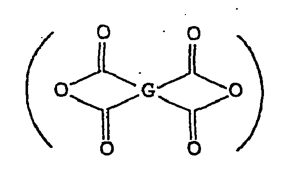 der Formel steht: 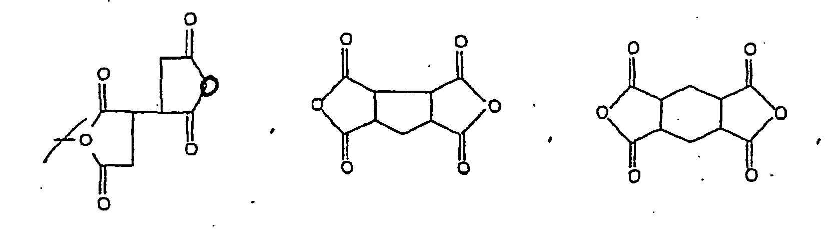 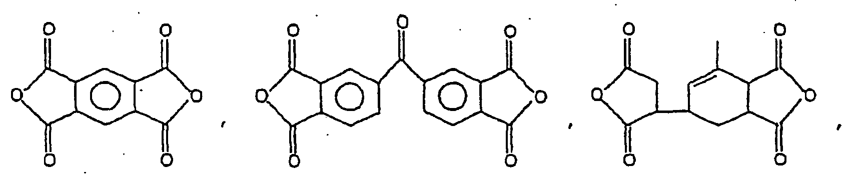 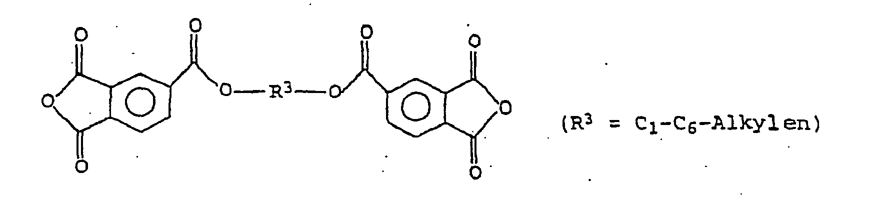 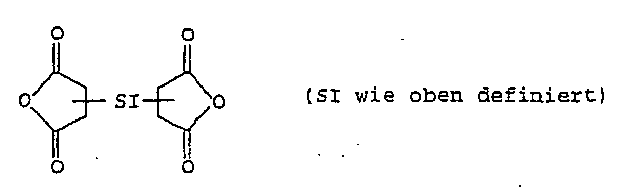
   Die Verbindungen der Formel VIII sind erhältlich durch Umsetzung eines polysiloxanhaltigen Diamins HR⁶N-SI-NR⁶H mit einem der Dianhydride in einem Lösungsmittel oder ohne Lösungsmittel bei einer Temperatur im Bereich von 20 bis 120°C. Dabei können bis zu 80 Mol-% des polysiloxanhaltigen Diamins durch ein Polyamiddiamin ersetzt werden. Das Molverhältnis von Dianhydrid zu Diamin liegt im Bereich von 0,5 bis 2.
   Zu (VIII) analoge Produkte kann man erhalten durch Umsetzung eines pblysiloxanhaltigen Dianhydrids der Struktur 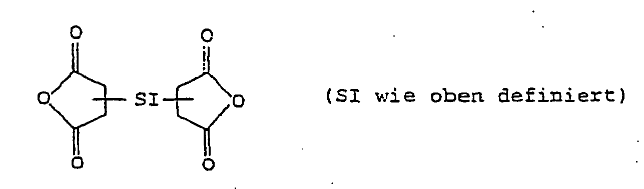 mit einem Diamin.
4. mehrwertige, siloxanhaltige Säuren, bei denen es sich um Polykondensate handelt, deren Ketten aus wenigstens einer Polysiloxaneinheit und wenigstens einer Polyurethaneinheit und/oder Polyharnstoffeinheit aufgebaut sind, wobei die Polyurethan- und/oder Polyharnstoffeinheiten Carbonsäure- und/oder Sulfonsäuregruppen aufweisen. Dabei handelt es sich insbesondere um Polyurethane oder Polyhamstoffe, die aus einem Diisocyanat und einem Diol oder Diamin hergestellt wurden, wobei das Molverhältnis NCO/acide Wasserstoffatome < 0,9 oder > 1,1 beträgt. Der K-Wert der Polymere (gemessen als 0,1 %ige Lösung in N-Methylpyrrolidon) ist vorzugsweise < 24. Derartige Polykondensate sind in der EP-A-636 361 beschrieben, auf deren Inhalt hiermit Bezug genommen wird.
   Vorzugsweise handelt es sich um Polykondensate mit Polysiloxaneinheiten der Formel

   ⁅(̵E―SI―E)̵CONH―R⁷―NHCO⁆ (IX)

   worin E für O oder NH steht, R⁷ für einen C₁-C₆-Alkylen oder Phenylenrest steht und SI die oben angegebenen Bedeutungen besitzt; und mit Polyurethan- und/oder Polyharnstoffeinheiten der Formel 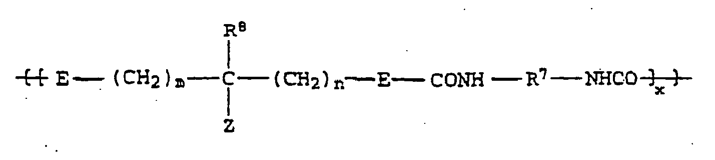 worin E für O oder NH steht, R⁷ für C₁-C₆-Alkylen oder Phenylen steht, R⁸ für oder C₁-C₄-Alkyl steht, Z für COOH oder SO₃H steht, m und n, die gleich oder verschieden sein können, für 1 bis 6 stehen und x für 1 bis 10 steht.

Als Amin sind alle kosmetisch akzeptablen Amine, die mit den obigen siloxanhattigen Säuren Salze bilden, brauchbar. Bevorzugt sind aliphatische Monoamine oder Polyamine, insbesondere Alkylamine, C₂-C₈-Alkylendiamine und - triamine, wobei die Alkylgruppen unabhängig voneinander vorzugsweise 1 bis 12 und insbesondere 1 bis 6 Kohlenstoffatome und gegebenenfalls 1 bis 6 Hydroxygruppen aufweisen. Besonders brauchbar sind Hydroxygruppen-haltige Amine. Beispiele für geeignete Amine sind Monoethanolamin, Diethanolamin, Triethanolamin, Dimethylethanolamin, Diethylethanolamin, Methyldiethanolamin, Ethyldiethanolamin, Butyldiethanolamin, 2-Amino-2-methylpropanol, 2-Amino-2-methylolpropanol und Polyamine, wie N-Methyldipropylentriamin oder N-Methylglucamin.

Bevorzugte siloxanhaltige Amine sind:
- einwertige, siloxanhaltige Amine der Formel

   R¹-NR⁹R¹⁰,

   worin R¹ die oben im Zusammenhang mit den Verbindungen der Formel IV angegebenen Bedeutungen besitzt und R⁹ und R¹⁰, die gleich oder verschieden sein können, für H, C₁-C₆-Alkyl oder C₁-C₆-Hydroxyalkyl stehen. Beispiele für derartige Amine sind solche der Formeln 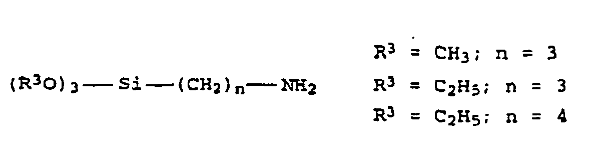 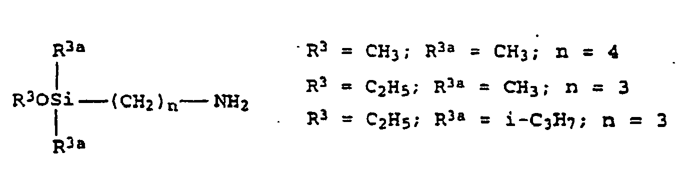 oder

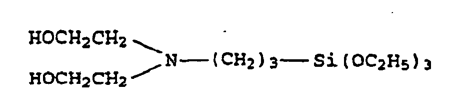

   (C₂H₅O)₃―Si―(CH₂)₃⁆NH

   Derartige Amine sind im Handel (Fa. Hüls) erhältlich.
- zweiwertige, slloxanhaltige Amine der Formel VI, worin E für NH₂ oder NHC₁-C₆-Alkyl steht. Beispiele für derartige Amine sind die Tegomer A-Si-Typen der Fa. Goldschmidt.
- mehrwertige, siloxanhaltige Amine, bei denen es sich um Polykondensate handelt, deren Ketten aus wenigstens einer Polysiloxaneinheit und wenigstens einer Polyurethaneinheit und/oder Polyharnstoffeinheit aufgebaut sind,
wobei die Polyurethan- und/oder Polyharnstoffeinheiten Amingruppen aufweisen. Derartige Polykondensate sind in der EP-A-636 361 beschrieben, auf deren Inhalt hiermit Bezug genommen wird.

Vorzugsweise handelt es sich um Polykondensate mit Polysiloxaneinheiten der Formel

⁅(̵E―SI―E)̵CONH―R⁷―NHCO⁆ (IX)

worin E für O oder NH steht, R⁷ für einen C₁-C₆-Alkylen- oder Phenylenrest steht und Sl die oben angegebenen Bedeutungen besitzt, und mit Polyurethan- und/oder Polyharnstoffeinheiten der Formel

[[E―(CH₂)ₘ―L―(CH₂)ₙ―E―CONH―R⁷―NHCO]ₓ] (X)

worin E für O oder NH steht, L für 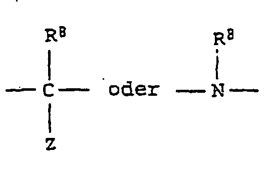
- steht,
   R⁷ für C₁-C₆-Alkylen oder Phenylen steht, R⁸ für H oder C₁-C₄-Alkyl steht, Z für NR¹¹R¹² steht, m' und n, die gleich oder verschieden sein können, für 1 bis 6 stehen, x für 1 bis 10 steht und R¹¹ und R¹², die gleich oder verschieden sein können, für C₁-C₆-Alkyl stehen.

Vorzugsweise ist das Molekulargewicht eines siloxanfreien Salzes kleiner als 3000 und insbesondere kleiner als 2000. Das Molekulargewicht siloxanhaftiger Salze kann bis zu 30000 betragen.

Besonders bevorzugte Salze sind die Salze von Isophthalsäure, 5-NaSO₃-Isophthalsäure. Trimcththsäure oder 1,3,5-Benzoltricarbonsäure mit 2-Amino-2-methylpropanol, sowie siloxanhaltige Salze, wobei folgende Typen bevorzugt sind:
- Salze aus einer einwertigen Säure mit einem einwertigen Amin, wobei mindestens eine der beiden Komponenten eine Siloxaneinheft aufweist;
- Salz aus einer einwertigen Säure mit einem mehrwertigen Amin, wobei mindestens eine der Komponenten eine Siloxaneinheit aufweist;
- Salz aus einer mehrwertigen Säure mit einem einwertigen Amin, wobei mindestens eine der Komponenten eine Siloxaneinheit aufweist;
- Salz aus einer zweiwertigen Säure mit einem zweiwertigen Amin, wobei mindestens eine der Komponenten eine Siloxaneinheit aufweist;

Gemäß einer bevorzugten Ausführungsform umfaßt das Mittel zusammen mit einem siloxanhaltigen Salz auch mindestens ein Silicon, vorzugsweise in einer Menge von 0,0001 bis 0,2 Gew.-%, bezogen auf das Gesarntgewicht des Mittels.

Besonders bevorzugt ist ein Mittel, das enthält:
a) 0,5 bis 15 Gew.% eines siloxanfreien Haarfestigerpolymers,
b) 0,1 bis 8 Gew.-% Salz der Formel I und
c) 0,0005 bis 0,15 Gew.-%, insbesondere 0,001 bis 0,1 Gew.-% Silicon.

Als Haartestigerpolymere sind insbesondere Polyurethane, Polyester, Polyamide, Poly(amid)ester Homo- und Copolymere von monoolefinisch ungesättigten Monomeren brauchbar, wobei diese Polymere ionogcnc b/w ionische, an die Polymerkette gebundene Gruppen aufweisen, damit die Polymere in Wasser löslich bzw. dispergierbar sind. Bei diesen Gruppen handelt es sich vorzugsweise um Carbonsäuregruppen und/oder Sulfonsäuregruppen und/oder stickstoffhaltige Gruppen (Amine) bzw. Carboxylatgruppen und/oder Sulfonatgruppen und/oder quaternisierte stickstoffhaltige Gruppen. Derartige Polymere sind beispielsweise beschrieben in der US-A-3,475,206 und 3 412.054 sowie in der DE-A-15 70 615. Vorzugsweise verwendet man jedoch die in der DE-A-42 25 045: DE-A-42 41 118 und EP-A-619111 sowie die in der DE-A-42 24 761 beschriebenen Polymere. Es handelt sich dabei um folgende Polymere:
1. In Wasser lösliche oder dispergierbare anionische Polyurethane aus
   a) mindestens einer Verbindung, die zwei oder mehrere aktive Wasserstoffatome pro Moleküle enthält,
   b) mindestens einem Säure- oder Salzgruppen enthaltenden Diol und
   c) mindestens einem Diisocyanat.
   die eine Glastemperaturvon mindestens 15°C und Säurezahlen im Bereich von 12 bis 150, vorzugsweise 30 bis 90, besitzen, und die Salze davon.
   Bei der Komponente a) handelt es sich insbesondere um Diole, Aminoalkohole, Diamine, Polyesterole, Polyetherole mit einem zahlenmittleren Molekulargewicht von jeweils bis zu 3000 oder deren Mischungen, wobei bis zu 3 Mol-% der genannten Verbindungen durch Triole oder Triamine ersetzt sein können. Bevorzugt sind Diole und Polyesterdiole. Insbesondere umfaßt die Komponente (a) mindestens 50 Gew.-%, bezogen auf das Gesamtgewicht der Komponente (a), eines Polyesterdiols. Als Polyesterdiole kommen alle diejenigen in Betracht, die üblichenueise zur Herstellung von Polyurethanen eingesetzt werden, insbesondere Umsetzungsprodukte aus Phthalsäure un Diethylenglycol, Isophthalsäure und 1 ,4-Butandiol, lsophlhalsäure/Adipinsäure und 1,6-Hexandiol sowie Adipinsäure und Ethylenglycol oder 5-NaSO₃-Isophthalsäure, Phthalsäure, Adipinsäure und 1,6-Hexandiol.
   Brauchbare Diole sind z.B. Ethylenglycol, Propylenglycol, Butylenglycol, Neopentylglycol, Polyetherole, wie Polyethylenglycole mit Molekulargewichten bis zu 3000, Blockcopolymerisate aus Ethylenoxid und Propylenoxid mit zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Bevorzugt sind Ethylenglycol, Neopentylglycol, Di-, Tri-, Tetra-, Penta oder Hexaethylenglycol. Brauchbare Diole sind außerdem Poly(α-hydroxycarbonsäure)diole.
   Geeignete Aminoalkohole sind z.B. 2-Aminoethanol, 2-(N-Methylamino)ethanol, 3-Aminopropanol oder 4-Aminobutanol.
   Geeignete Diamine sind z.B. Ethylendiamin, Propylendiamin, 1,4-Diaminobutan und 1,6-Diaminohexan sowie α.ω-Diamine, die durch Aminierung von Polyalkylenoxiden mit Ammoniak herstellbar sind.
   Bei der Komponente b) handelt es sich insbesondere um Dimethylolpropansäure oder Verbindungen der Formeln 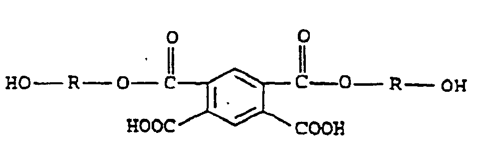 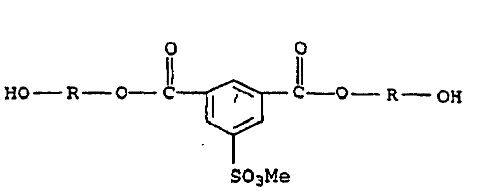 worin R jeweils für eine C₂-C₁₈-Alkylengruppe steht und Me für Na oder K steht.
   Bei der Komponente c) handelt es sich insbesondere um Hexamethylendiisocyanat, Isophorondiisocyanat, Methyldiphenyllsocyanat (MDI) und/oder Toluylendüsocyanat.
   Die Polyurethane sind dadurch erhältlich, daß man die Verbindungen der Gruppen a) und b) unter einer Inertgasatmosphäre in einem inerten Lösemittel bei Temperaturen von 70 bis 130°C mit den Verbindungen der Gruppe c) umsetzt. Diese Umsetzung kann gegebenenfalls in Gegenwart von Kettenverlängem durchgeführtwerden, um Polyurethane mit höheren Molekulargewichten herzustellen. Wie bei der Herstellung von Polyurethanen üblich, werden die Komponenten [(a)+(b)]:(c) im molaren Verhältnis von 0,8 bis 1,1:1 eingesetzt. Die Säurezahl der Polyurethane wird von der Zusammensetzung und der Konzentration der Verbindungen der Komponente (b) in der Mischung aus den Komponenten (a)+(b) bestimmt. Die Polyurethane haben K-Werte nach H. Fikentscher (bestimmt in 0,1 gew.-%igen Lösungen in N-Methylpyrrolidon bei 25°C und PH 7) von 15 bis 100, vorzugsweise 25 bis 50.
   Die Säuregruppen enthaltenden Polyurethane sind nach Neutralisation (teilweise oder vollständig) wasserlöslich bzw. ohne Zuhilfenahme von Emulgatoren dispergierbar. In aller Regel weisen die Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Besonders haben sich zur Neutralisation der Säuregruppen enthaltenden Polyurethane 2-Amino-2-Methylpropanol, Diethylamlnopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 bis 40 % oder vollständig, d.h. zu 100% erfolgen.
   Diese Polymere und ihre Herstellung sind in der DE-A-42 25 045 näher beschrieben, auf die hiermit in vollem Umfang Bezug genommen wird.
2. In Wasser lösliche oder dispergierbare, kationische Polyurethane und Polyharnstoffe aus
   a) mindestens einem Diisocyanat, welches bereits vorher mit einer oder mehreren Verbindungen, die zwei oder mehrere aktive Wasserstoffatome pro Molekül enthalten, umgesetzt worden sein kann, und
   b) mindestens einem Diol, primären oder sekundären Aminoalkohol, primären oder sekundären Diamin oder primären oder sekundären Triamin mit einem oder mehreren tertiären, quartären oder protonierten tertiären Aminstickstoffatomen,
   die eine Glasübergangstemperatur von mindestens 25°C und eine Aminzahl von 50 bis 200, bezogen auf die nicht-quatemisierten oder protonierten Verbindungen, aufweisen, und die Salze davon. Die Aminzahl liegt vorzugsweise im Bereich von 65-180, insbesondere 70 bis 170, besonders bevorzugt 75 bis 160 und ganz besonders 80 bis 150.
   Bevorzugte Diisocyanate sind wie oben unter 1) angegeben. Verbindungen mit zwei oder mehreren aktiven Wasserstoffatomen sind Diole, Aminoalkohole, Diamine, Polyesterole. Polyamiddiamine und Polyetherole. Geeignete Verbindungen dieser Art sind wie oben unter 1) angegeben.
   Die Herstellung der Polyurethane erfolgt wie oben unter 1) beschrieben. Geladene kationische Gruppierungen lassen sich aus den vorliegenden tertiären Aminstickstoffatomen entweder durch Protonierung, z.B. mit Carbonsäuren wie Milchsäure, oder durch Quaternisierung, z.B. mit Alkylierungsmitteln wie C₁- bis C₄-Alkylhalogeniden oder-sulfaten in den Polyharnstoffen erzeugen. Beispiele solcher Alkylierungsmittel sind Ethylchlorid, Ethylbromid, Methylchlorid, Methylbromid, Dimethylsulfat und Diethylsulfat.
   Diese Polymere und ihre Herstellung sind in der DE-A-42 41 118 näher beschrieben, auf die hiermit in vollem Umfang Bezug genommen wird.
3. Lineare Polyurethane mit Carboxylatgruppen aus
   a) einer 2,2-Hydroxymethyl-substituierten Carbonsäure der Formel 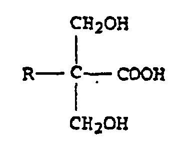 worin R für ein Wasserstoffatom oder eine C₁-C₂₀-Alkylgruppe steht, die in einer Menge verwendet wird, welche ausreicht, daß in dem Polyurethan 0,35 bis 2,25 Milliäquivalente Carboxylgruppen pro g Polyurethan vorhanden sind,
   b) 10 bis 90 Gew.-%, bezogen auf das Gewicht des Polyurethans, einer oder mehrerer organischer Verbindungen mit nicht mehr als zwei aktiven Wasserstoffatomen und
   c) einem oder mehreren organischen Diisocyanaten.
      Die im Polyurethan enthaltenden Carboxylgruppen werden abschließend mit einer geeigneten Base zumindest teilweise neutralisiert. Diese Polymere und ihre Herstellung sind in der EP-A-619 111 beschrieben, auf die hiermit in vollem Umfang Bezug genommen wird.
4. Carboxylhaltige Polykondensationsprodukte mit Glastemperaturen von >20°C aus Anhydriden von Tri- oder Tetracarbonsäuren und Diolen, Diaminen oder Aminoalkoholen (Polyester, Polyamide oder Polyesteramide). Diese Polymere und ihre Herstellung sind in der DE-A-42 24 761 näher beschrieben, auf die hiermit in vollem Umfang Bezug genommen wird.
5. Polyacrylate und Polymethacrylate, wie sie in den DE-A-43 14 305, 36 27 970 und 2917 504 näher beschrieben sind. Auf diese Publikatiionen wird hiermit in vollem Umfang Bezug genommen.

Weitere geeignete Haarfestigerpolymere sind:
- nicht-ionische, wasserlösliche bzw. wasserdispergierbare Polymere oder Oligomere, wie Polyvinylcaprolactam, z.B. Luviskol Plus (BASF) oder Polyvinylpyrrolidon und deren Copolymere, insbesondere mit Vinylestem, wie Vinylacetat, z.B. Luviskol VA 37 (BASF); Polyamide. z.B. auf Basis von Itaconsäure und aliphatischen Diaminen;
- amphotere oder zwitterionische Polymere, wie die unter den Bezeichnungen Amphomer® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973. DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind.
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylatcopolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer. Acrylsäure/Ethylacrylat/N-tert.Butylacrylamid-Terpolymere, die beispielsweise unter der Bezeichnung Ultrahold® strong (BASF) vertrieben werden; und Luvimer® (BASF, Terpolymer aus t-Butylacrylat/Ethylacrylat und Methacrylsäure) oder
- kationische (quatemisierte) Polymere, z.B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinyfpyrrolidon mit quatemären Ammoniumverbindungen entstehen), Polymer JR (Hydroxyethylcellulose mit kationischen Gruppen), Polyquaternium Typen (CTFA-Bezeichnungen) etc.

Die erfindungsgemäß zur Anwendung kommenden Polymere besitzen vorzugsweise einen K-Wert von 25 bis 100, bevorzugt 25 bis 50. Die Polymere sind in dem erfindungsgemäßen Mittel im allgemeinen in einer Menge im Bereich von 0,2 bis 20 Gew.-%, bezogen auf das Gesamtgewicht des Mittels, enthalten. Das Salz kommt in einer zur Verbesserung der Auswaschbarkeit der Polymeren wirksamen Menge zur Anwendung. Im allgemeinen setzt man das Salz in einer Menge von 0,02 bis 10 Gew.-%, und vorzugsweise von 0,1 bis 8 Gew.-%, bezogen auf das Gesamtgewicht des Mittels, ein.

Das Gewichtsverhältnis von Komponente (a) zu Komponente (b) liegt im allgemeinen im Bereich von 1 0.01 bis 1 : 1,0, vorzugsweise 1 : 0,02 bis 1 : 0,5.

Die erfindungsgemäß eingesetzten Salze verbessern die Auswaschbarkeit der Haarfestigerpolymere und deren Filmeigenschaften. Sie verleihen den mit den erfindungsgemäßen Mitteln behandelten Haaren Glanz und Geschmeidigkeit und verringern die Klebrigkeit der Haarfestigerpolymere. Außerdem sind die erfindungsgemäßen Carbonsäuren in der Kosmetik, Pharmazie, Textil- und Papierindustrie als Additive, insbesondere als Lösungsvermittler, Tenside, Emulgatoren oder Schutzkolloide und zur Verbesserung der Oberflächeneigenschaften von Filmen denen sie Glätte und Glanz verleihen, brauchbar. Dies gilt insbesondere für die siloxanhaltigen Säuren bzw. Basen. Durch deren Anwendung können Haarbehandlungsmittel mit bis zu 0,2 Gew.-%, bezogen auf das Gesamtgewicht des Mittels, eines Silicons formuliert werden. Die erhaltenen Filme sind glatt, klar und gut auswaschbar.

Die erfindungsgemäßen Haarbehandlungsmittel liegen üblicherweise in Form einerwäßrigen Dispersion oder in Form einer alkoholischen oder wäßrig-alkoholischen Lösung vor. Beispiele geeigneter Alkohole sind Ethanol. Propanol, Isopropanol etc.

Weiter enthalten die erfindungsgemäßen Haarbehandlungsmittel im allgemeinen übliche kosmetische Hilfsstoffe, beispielsweise Weichmacher, wie Glycerin und Glycol; Emollienzien, Gleitmittel und Penetrationsmittel wie Lanolinverbindungen; Parfüms; UV-Absorber; Farbstoffe; Verdickurigsmittel; antistatische Mittel; Mittel zur Verbesserung der Kämmbarkeit; Konservierungsmittel; und Schaumstabilisatoren. Die Mittel können auch mindestens ein anderes Haarfestigerpolymer enthalten.

Wenn die erfindungsgemäßen Mittel als Haarspray formuliert sind, enthalten sie eine ausreichende Menge eines Treibmittels, beispielsweise ein niedrigsiedender Kohlenwasserstoffoder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel wird so gering wie möglich gehalten, um den VOC-Gehalt nicht unnötig zu erhöhen. Sie beträgt im allgemeinen nicht mehr als 40 Gew.-%, bezogen auf das Gesamtgewicht des Mittels.

Die erfindungsgemäßen Mittel besitzen den Vorteil, daß sie einerseits den Haaren Festigkeit, Glanz und Geschmeidigkeit verleihen und andererseits die in den Mitteln enthaltenen Polymere leichter auswaschbar (redispergierbar)sind. Darüber hinaus lassen sich Mittel mit einem VOC-Gehalt von weniger als 60 Gew.% und auch rein wäßrige Formulierungen herstellen, selbst wenn sie als Haarspray formuliert sind.

Die nachfolgenden Beispiele erläutern die Erfindung:

### Beispiel 1

### Polyurethanherstellung:

In einem Vierhalskolben, der mit Rührer, Tropftrichter, Thermometer, Rückflußkühler und einer Vorrichtung für das Arbeiten unter Stickstoff ausgestattet war, wurden 0,5 Mol Polyesterdiol (M_{W} = 1000 g/mol), hergestellt aus Isophthalsäure, Adipinsäure und Hexandiol, 0,6 Mol Diethylenglycol und 1,25 Mol Dimethylolpropansäure in Methylethylketon (ca. 50%-ige Lösung) unter Erhitzen auf eine Temperatur von 80°C und unter Rühren gelöst. Sobald sich alles gelöst hatte, wurde das Reaktionsgemisch auf ca. 50°C abgekühlt. Anschließend wurden unter Rühren 2.5 Mol Isophorondiisocyanat zugetropft, wobei die Reaktionstemperatur anstieg. Bei Rückflußtemperatur wurde das Reaktionsgemisch dann solange gerührt, bis der Isocyanatgruppengehalt des Gemisches praktisch konstant blieb. Die restlichen Isocyanatgruppen wurden durch Zusatz eines Amins, z.B. 2-Amino-2-methyl-1-propanol inaktiviert. Freie COOH-Gruppen wurden mit 2-Amino-2-methylpropanol neutralisiert. Anschließend wurde Wasser zugegeben und der größte Teil des Methylethylketons unter vermindertem Druck bei ca. 40°C entfernt. Man erhielt eine Dispersion des Polyurethans *(K-Wert 27)*, die für die in dem nachfolgenden Beispiel 2 beschriebenen Versuche verwendet wurde.

### Beispiel 2

### Auswaschbarkeit der Polymere:

Die Auswaschbarkeit des gemäß Beispiels 1 erhaltenen Polymers mit Wasser wurde untersucht. Zu diesem Zweck wurde ein Film des Polymers aus 5%igerwäßriger oder wäßriger-alkoholischer Formulierung (1:1 V/V) auf einer Glasplatte erzeugt, indem die Polymerdispersion auf die Glasplatte gegossen wurde. Man ließ 20 h bei Raumtemperatur trocknen. Die Auswaschbarkeit (Redispergierbarkeit) des Films mit Wasser wurde durch Rubbein per Finger bestimmt. Im Versuch 1 wurde kein Salz zugesetzt, In den Versuchen 2 bis 4 wurden dagegen 5 Gew.-% des Polymers ersetzt durch eines der folgenden Salze:
A. Salz aus 1 Mol Isophthalsäure und 2 Mol 2-Amino-2-methylpropanol;
B. Salz aus 1 Mol 5-NaSO₃-Isophthalsäure und 2 Mol 2-Amino-2-methylpropanol;
C. Salz aus 1 Mol 1,3,5-Benzottricarbonsäure und 3 Mol 2-Amino-2-methylpropanol.

Die erhaltenen Ergebnisse sind in der nachfolgenden Tabelle angegeben.

**Tabelle 1:**

| Auswaschbarkeit der Polymere | | | | |
|---|---|---|---|---|
| Versuch | K-Wert des Polyurethans | Salz | Auswaschbarkeit | |
| | | | Film aus wäßriger Dispersion | Film aus H₂O/EtOH-Dispersion |
| 1 | 27 | - | schlecht | schlecht |
| 2 | 27 | A | gut | gut |
| 3 | 27 | B | gut | gut |
| 4 | 27 | C | gut | gut |

Es ist ersichtlich, daß die mit den erfindungsgemäßen Mitteln erhaltenen Filme überraschenderweise besser auswaschbar sind als der mit dem Mittel des Standes des Technik erhaltene Film.

### Beispiel 3

### Haarspray-Formulierungen mit einem VOC-Gehalt von 55 Gew.-%:

| | Formulierung mit Salz | ohne Salz |
|---|---|---|
| Polyurethan gemäß Beispiel 1 | 4,75 Gew.-% | 5 Gew.-% |
| Wasser | 40 Gew.-% | 40 Gew.-% |
| Ethanol | 25 Gew.-% | 25 Gew.-% |
| Dimethylether | 30 Gew.-% | 30 Gew.-% |
| Salz A, B oder C | 0,25 Gew.-% | - |
| Parfüm q.s. | | |

Die Auswaschbarkeit der mit diesen Formulierungen erhaltenen Filme wurde an künstlichen Modellköpfen wie folgt bestimmt:

Das Haarspray wurde in einem Sprühvorgang von 10 sec (Auftragsmenge ca. 2.5 g) auf die Haare der Modellköpfe aufgetragen. Nach zweistündigem Trocknen im Klimaraum (Luftfeuchtigkeit 45%; Temperatur 20°C) wurde die Festigungswirkung beurteilt. Dieser Vorgang wurde insgesamt 3x wiederholt. Der besprühte Modellkopf wurde im Klimaraum über Nacht getrocknet. Danach wurde mit Texapon NSO nicht länger als 5 min shampooniert und gewaschen. Nach dem Trocknen wurde die Auswaschbarkeit von geschulten Fachleuten beurteilt. Es wurde gefunden, daß der mit dem erfindungsgemäßen Mittel (Formulierung mit Salz) erhaltene Film gut auswaschbar war, während sich der mit dem Mittel ohne Salz erhaltene Film nur schlecht auswaschen ließ.

### Beispiel 4

a) Herstellung eines Salzes einer Carbonsäure der Formel VII (wenn (n1 + n2 = 0):
   93 g (0,1 mol) Tego OF 2010 (Polydimethylsiloxan-diamin der Fa. Goldschmidt) wurden zusammen mit 26 g (0,2 mol) Itaconsäure in 45 g Ethanol:Wasser (1:1) unter Rücklußtemperatur2h gerührt. Nach dem Abdestillieren von Ethanol und Wasser wurde das Reaktionsgemisch bei einer Temperatur von etwa 140-170°C noch 2h gehalten. Man erhielt eine gelbe, zähe Masse, die in Ethanol gut löslich ist und nach Neutralisation mit 2 Mol 2-Amino-2-methylpropanol in Wasser eine stabile Dispersion bildet.
b) Herstellung eines Salzes einer Carbonsäure der Formel VII (wenn R⁵ = -CH₂CH₂-; n1 + n2 = 5):
   1 Mol Tego OF 2010 wurde mit 7 Mol Itaconsäure in 450 g Ethanol: Wasser (1:1) 2h unter Rückfluß und unter Stickstoff gerührt Nach dem Abdestillieren von Ethanol und Wasser wurde das Reaktionsgemisch nach 2h bei einer Temperatur von 140-170°C gehalten. Anschließend wurden 5 Mol Ethylendiamin in Wasser (1:1) zugegeben und die obige Behandlung wurde wiederholt. Man erhielt ein hartes gelbes Produkt, das in Ethanol gut löslich ist und nach Neutralisation mit 2-Amino-2-methylpropanol in Wasser eine stabile Dispersion bildet

### Beispiel 5

### Herstellung eines Salzes einer Carbonsäure der Formel VIII:

83 g (0,1 mol) Tego OF 2010 (Polydimethylsiloxan-diamin der Fa. Goldschmidt) wurden zusammen mit 29 g (0,09 mol) 3,3',4,4'-Benzophenon-tetracarbonsäuredianhydrid (BPDA) in 50 g Ethanol:Wasser (1:1) bei RT ca. 1 h gerührt. Das Reaktionsgemisch wurde danach bei einer Temperatur von etwa 40-70°C 3 h unter Rühren gehalten. Man erhielt nach Neutralisation mit Aminomethylpropanoi eine klare gelbe Lösung, welche nach Trocknen bei 120°C/Vakuum ein elastisches Produkt ergab. Das Produkt löst sich in Ethanol und es bildet sich eine sehr stabile wäßrige Dispersion.

### Beispiel 6

### Herstellung eines Salzes aus einer einwertigen Carbonsäure und einem zweiwertigen Amin

93 g (0,1 mol) Tegomer A-Si 2120 (Polydimethylsiloxandiamin, etwa 930) wurden mit 20 g 90%iger Milchsäure (0,2 mol) neutralisiert.

Das erhaltene Salz wurde in 330 g Ethanol gelöst. Anschließend wurden 0,44 g Abil 200 (Polydimethylsiloxan der Fa. Goldschmidt) zugegeben. Man erhielt fast klare, stabile Dispersion.

### Beispiel 7

Die Eigenschaften eines Polymerfilms wurden bestimmt, wobei der Polymerfilm aus Ethanol/H₂O(8/2 V/V) erzeugt wurde. Die Auswaschbarkeit wurde wie In Beispiel 2 bestimmt. Die Klarheit wurde visuell und die Glätte des Films wurde von erfahrenen Prüfem durch Reiben mit den Fingern bestimmt. In den Versuchen 1, 2, 7 und 8 wurde kein Salz zugesetzt, in den Versuchen 3 bis 6, 9 und 10 wurde dagegen ein Teil des Polymers ersetzt durch das Salz der Beispiele 4 bis 6. In den Versuchen 2 und 5 bis 10 wurden der Dispersion außerdem 0,0005 bzw. 0,001 Gew.-% Siliconöl zugesetzt. Die Ergebnisse sind in der nachfolgenden Tabelle 2 angegeben.

**Tabelle 2: Eigenschaften des erhaltenen Polymerfilms**

| Versuch Nr. | Polymer Gew.-%⁴¹ | Salz | | Siliconöl Gew.-% | Filmeigenschaften | | |
|---|---|---|---|---|---|---|---|
| | | Beispiel | Gew.-% | | Glätte | Klarheit | Auswaschbarkeit |
| 1 | 5⁴⁾ | - | - | - | mäßig | klar | schlecht |
| 2 3 | 5⁴⁾ 3⁴⁾ | 4a | 2 | 0,0005 | glatt glatt | trüb | schlecht |
| | | | | | | klar | gut |
| 4 | 3⁴⁾ | 5 | 2 | - | glatt | klar | gut |
| 5 | 3⁴⁾ | 5 | 2 | 0,001 | sehr glatt | klar | gut |
| 6 | 4,5⁴⁾ | 6¹⁾ | 0,5 | 0,002 | sehr glatt | klar | auswaschbar |
| 7 | 5 ²⁾ | - | - | 0,0005 | glatt | leicht trüb | schlecht |
| 8 | 5 ³⁾ | - | - | 0,0005 | glatt | leicht trüb | auswaschbar |
| 9 | 4,5 ²⁾ | 6¹⁾ | 0,5 | 0,002 | sehr glatt | klar | auswaschbar |
| 10 | 4,5 ³⁾ | 6 | 0,5 | 0,002 | sehr glatt | klar | auswaschbar |

| | | | | | | | |
|---|---|---|---|---|---|---|---|
| *) Polyurethan gemäß Beispiel 1 wurde für Versuch Nr. 1 - 6 verwendet | | | | | | | |
| ¹⁾ Die Formulierung wurde hergestellt aus 4,5 g mit AMP neutralisiertem Polymer und 2 g der Lösung gemäß Beispiel 6 (entspricht 0.5 g Salz + 0,002 g Siliconöl in 1,5 g Ethanol). Danach mit 20 g Wasser und 78 g Ethanol aufgelöst. | | | | | | | |
| ²⁾ Luvimer® 100P: Terpolymer aus t-Butylacrylat, Ethylacrylat und Methacrylsäure (BASF) | | | | | | | |
| ³⁾ Luviskol® Plus*: Polyvinylcaprolactam (BASF) | | | | | | | |
| ⁴⁾ Polymer gemäß Beispiel 1 AMP = 2-Amino-2-methyl-l-propanol | | | | | | | |

## Patentansprüche

1. Haarbehandlungsmittel, enthaltend:
a). mindestens ein in Wasser oder einem Gemisch aus Wasser und einem C₁-C₄-Alkohol lösliches oder dispergierbares Haarfestigerpolymer und
b). mindestens ein in Wasser oder einem Gemisch aus Wasser und einem C₁-C₄-Alkohol lösliches oder dispergierbares Salz der Formel I:
[A-(X)ₙ]ⁿ⁻ · HB⁺ I
worin A für einen kosmetisch akzeptablen, aromatischen Rest steht, der 1, 2 oder 3 Substituenten aufweisen kann, die gleich oder verschieden sein können und ausgewählt sind unter C₁-C₆-Alkyl, C₁-C₆-Alkoxy, Mono- oder Polyhydroxy-C₁-C₆-alkyl, Hydroxy und Amino,
X für eine Carboxylat gruppe steht;
B eine kosmetisch akzeptable Aminbase
n für 2 oder 3 steht; und
wobei die Aminbase B
ausgewählt ist unter Di-C₁-C₆-alkyl-ethanolaminen, Mono-C₁-C₆-alkyl-diethanolaminen, Monoethanolamin, Diethanolamin, Triethanolamin, 2-Amino-2-methylpropanol und 2-Amino-2-methylolpropanol.

2. Haarbehandlungsmittel, enthaltend:
a). mindestens ein in Wasser oder einem Gemisch aus Wasser und einem C₁-C₄-Alkohol lösliches oder dispergierbares Haarfestigerpolymer und
b). mindestens ein in Wasser oder einem Gemisch aus Wasser und einem C₁-C₄-Alkohol lösliches oder dispergierbares Salz der Formel I:
[A-(X)ₙ]ⁿ⁻ · [HₘB]^{m+} I
worin A für einen kosmetisch akzeptablen aliphatischen, cycloaliphatischen oder aromatischen Rest steht, der siloxan haltige Einheiten und/oder 1, 2 oder 3 Substituenten aufweisen kann, die gleich oder verschieden sein können und ausgewählt sind unter Cₗ-C₆-Alkyl, C₁-C₆-Alkoxy, Mono- oder Polyhydroxy-C₁-C₆-alkyl, Hydroxy und Amino, wobei gegebenenfalls die Kette des aliphatischen Restes durch bis zu 30 -CONH-Gruppen unterbrochen sein kann und der Ring des cycloaliphatischen Restes eine -CO-N-Gruppe aufweisen kann, wobei zwei cycloaliphatische Reste jeweils am N-Atom über eine C₂-C₈-Alkylen- oder eine p-Xylylengruppe verbunden sind;
X für eine Carboxylat-, Sulfonat-, Phosphat- oder Phosphonatgruppe steht;
B eine kosmetisch akzeptable Aminbase bedeutet die siloxan haltige Einheiten aufweisen kann;
n für 1 bis 30 steht; und
m die Wertigkeit des Amins B bedeutet, wobei
A und/oder B eine Siloxaneinheit aufweist.

3. Haarbehandlungsmittel nach Anspruch 1, **dadurch gekennzeichnet, daß** das Salz gebildet ist aus Isophthalsäure, 5-NaSO₃-Isophthalsäure, 1,2,4-Benzoltricarbonsäure oder 1,3,5-Benzoltricarbonsäure und 2-Amino-2-methylpropanol.

4. Haarbehandlungsmittel nach Anspruch 2, **dadurch gekennzeichnet, daß** es zusätzlich ein wasserunlösliches Silicon, insbesondere Dimethicon, enthält.

5. Haarbehandlungsmittel nach Anspruch 4, **dadurch gekennzeichnet, daß** es
a) ein siloxanfreies Haarfestigerpolymer,
b) ein Salz der Formel I,
c) ein wasserunlösliches Silicon
enthält.

6. Haarbehandlungsmittel nach Anspruch 2, 4 oder 5, **dadurch gekennzeichnet, daß** das Salz gebildet ist aus einer siloxanhaltigen Säure der Formeln
R¹―NHCO―R²―COOH (IV) 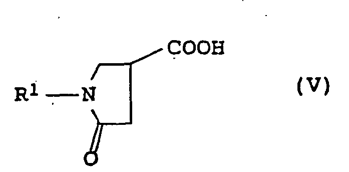
worin R¹ für 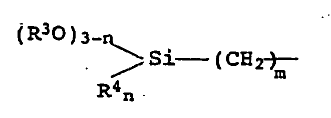 mit n = 0=3
und m = 1-6
steht, wobei R³ und R⁴ für Cₗ-C₆-Alkyl stehen und R² für einen der folgenden Reste steht:
R² = ―(CH₂)̵ₘ mit m = 2. - 6
--CH = CH―

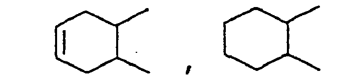

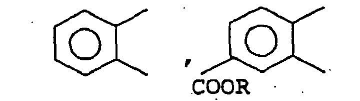

7. Haarbehandlungsmittel nach Anspruch 2, **dadurch gekennzeichnet, daß** das Salz gebildet ist aus einem siloxanhaltigen, einwertigen Amin der Formel R¹-NR⁹R¹⁰,
worin R¹ die in Anspruch 6 angegebenen Bedeutungen besitzt und _{R}⁹ und R¹⁰, die gleich oder verschieden sein können, für H, Cₗ-C₆-Alkyl oder C₁-C₆-Hydroxyalkyl stehen.

8. Haarbehandlungsmittel nach Anspruch 2, **dadurch gekennzeichnet, daß** das Salz gebildet ist aus einer siloxanhaltigen, zweiwertigen Säure der Formel (VI) oder (VII) 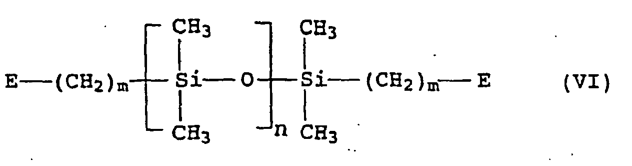 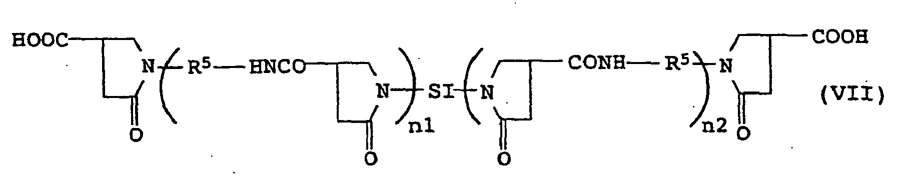 worin E für COOB oder 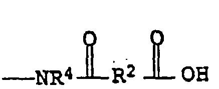 steht, wobei R² die oben angegebenen Bedeutungen besitzt und R⁴ für H oder C₁-C₆-Alkyl steht; m für 1 bis 6 und n für 1 bis 50 steht, R⁵ für C₂-C₄-Alkylen steht, n1 + n2 = 0-20 und SI für 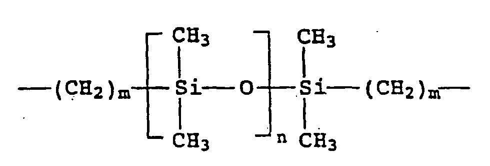 steht, wobei m für 1 bis 6 und n für 1 bis 50 steht.

9. Haarbehandlungsmittel nach Anspruch 2, **dadurch gekennzeichnet, daß** das Salz gebildet ist.aus einem siloxanhaltigen, zweiwertigen Amin der Formel (VI) 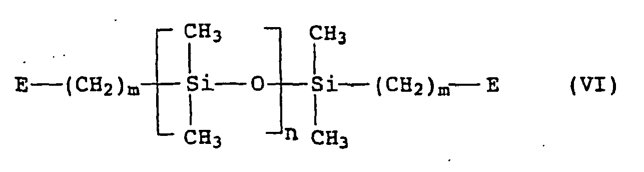 worin -E für -NH₂ oder NHC₁-C₆-Alkyl steht, m für 1-6 und n für 1-50 steht.

10. Haarbehandlungsmittel nach Anspruch 2, **dadurch gekennzeichnet, daß** das Salz gebildet ist aus einer siloxanhaltigen, mehrwertigen Säure der Formel (VIII) 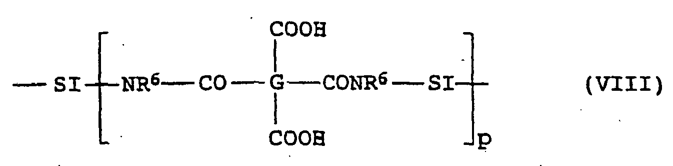 worin
R⁶ für H oder C₁-C₆-Alkyl steht, SI für die in Anspruch 8 angegebenen Bedeutungen steht, p für 1 bis 100 steht und G für den tetravalenten Rest eines Dianhydrids 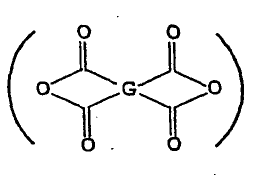 der Formel steht: 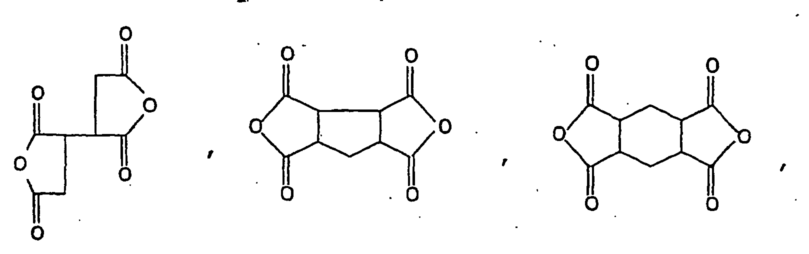 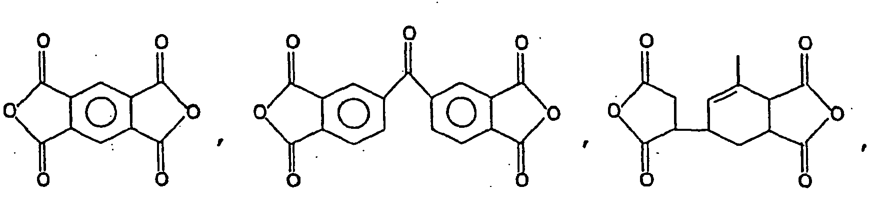 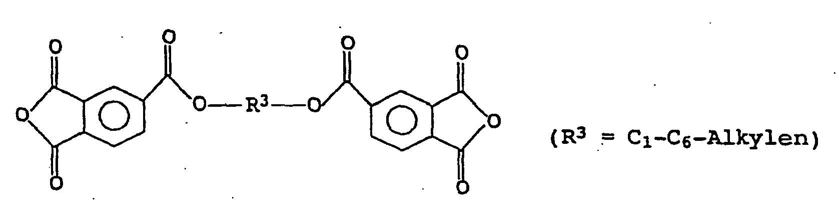 oder aus Polykondensaten mit Polysiloxaneinheiten der Formel
⁅(̵E―SI―E)̵CONH―R⁷―NHCO⁆ (IX)
worin E für O oder NH steht, R⁷ für einen C₁-C₆-Alkylen oder Phenylenrest steht und SI die oben angegebenen Bedeutungen besitzt, und mit Polyurethan- und/oder **Polyharnstoffeinheiten** der Formel 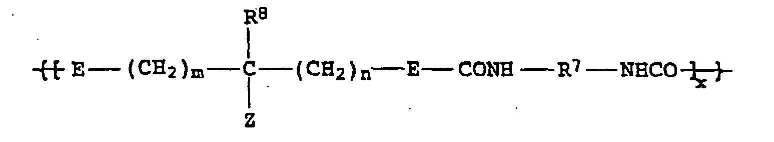 worin E für 0 oder NH steht, R⁷ für C₁-C₆-Alkylen oder Phenylen steht, R⁸ für H oder C₁-C₄-Alkyl steht, Z für COOH oder SO₃H steht, m und n,die gleich oder verschieden sein können, für 1 bis 6 stehen und x für 1 bis 10 steht.

11. Haarbehandlungsmittel nach Anspruch 2, **dadurch gekennzeichnet, daß** das Salz gebildet ist aus einem siloxanhaltigen, mehrwertigen Amin der in Anspruch 9 definierten Formel VI oder aus Polykondensaten mit Polysiloxaneinheiten der Formel
⁅(̵E―SI―E)̵CONH―R⁷―NHCO⁆ (IX)
worin E für O oder NH steht, R⁷ für einen C₁-C₆-Alkylen- oder Phenylenrest steht und SI die oben angegebenen Bedeutungen besitzt, und mit Polyurethan- und/oder Polyharnstoffeinheiten der Formel
[[E―(CH₂)ₘ―L―(CH₂)ₙ―E―CONH―R⁷―NHCO]ₓ] (X)
worin E für O oder NH steht, 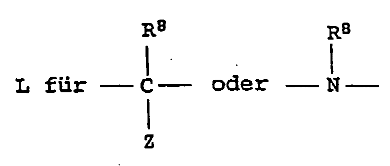 steht
_{R}⁷ für C₁-C₆-Alkylen oder Phenylen steht, R⁸ für H oder C₁-C₄-Alkyl steht, Z für NR¹¹R¹² steht, m und n, die gleich oder verschieden sein können, für 1 bis 6 stehen, x für 1 bis 10 steht und R¹¹ und R¹², die gleich oder verschieden sein können, für C₁-C₆-Alkyl stehen.

12. Haarbehandlungsmittel nach Anspruch 4, **dadurch gekennzeichnet, daß** es, jeweils bezogen auf das Gesamtgewicht des Mittels, enthält:
a) 0,5 bis 15 Gew.-% eines siloxanfreien Haarfestigerpolymers,
b) B) 0 ,1 bis 8 Gew. -% salz der Formel I und
c) 0,0005 bis 0,15 Gew.-%, insbesondere 0,001 bis 0,1 Gew.-% Silicon.

13. Verwendung eines Salzes der Formel I, wie in einem der Ansprüche 1 bis 11 definiert, als Hilfsmittel in der Kosmetik oder Pharmazie, insbesondere zur Verbesserung der Auswaschbarkeit von Haarfestigerpolymeren, als Tensid, Emulgator oder Schutzkolloid.

14. Verwendung der in einem der Ansprüche 2 und 4 bis 10 definierten, polysiloxanhaltigen Salze als Lösungsvermittler für hydrophobe Substanzen, insbesondere Silicone, oder zusammen mit einem Silicon als Hilfsmittel in der Kosmetik oder Pharmazie.

15. Carbonsäuren der in Anspruch 8 definierten Formel VII und deren Salze, insbesondere mit einer kosmetisch akzeptablen Aminbase, die siloxan- und/oder fluorhaltige Einheiten aufweisen kann.

## Claims

1. A hair treatment composition comprising:
a) at least one hairsetting polymer dispersible or soluble in water or in a mixture of water and a C₁-C₄ alcohol and
b) at least one salt which is dispersible or soluble in water or in a mixture of water and a C₁-C₄ alcohol and is of the formula I:
[A-(X)ₙ]ⁿ⁻. HB⁺ I
where A is a cosmetically acceptable, aromatic radical which may have 1, 2 or 3 substituents which may be identical or different and are selected from C₁-C₆-alkyl, C₁-C₆-alkoxy, mono- or poly-hydroxy-C₁-C₆-alkyl, hydroxyl and amino,
X is a carboxylate group;
B is a cosmetically acceptable amine base;
n is 2 or 3; and
the amine base B being selected from di-C₁-C₆-alkyl-ethanolamines, mono-C₁-C₆-alkyl-diethanolamines, monoethanolamine, diethanolamine, triethanolamine, 2-amino-2-methylpropanol and 2-amino-2-methylolpropanol.

2. A hair treatment composition comprising:
a) at least one hairsetting polymer dispersible or soluble in water or in a mixture of water and a C₁-C₄ alcohol and
b) at least one salt which is dispersible or soluble in water or in a mixture of water and a C₁-C₄ alcohol and is of the formula I:
[A-(X)ₙ]ⁿ⁻ · [HₘB]^{m+} I
where A is a cosmetically acceptable, aliphatic, cycloaliphatic or aromatic radical which may contain siloxane-containing units and/or may have 1, 2 or 3 substituents which may be identical or different and are selected from C₁-C₆-alkyl, C₁-C₆-alkoxy, mono- or poly-hydroxy-C₁-C₆-alkyl, hydroxyl and amino, it being possible if desired for the chain of the aliphatic radical to be interrupted by up to 30 -CONH groups and for the ring of the cycloaliphatic radical to have a -CO-N group, with two cycloaliphatic radicals being attached in each case to the nitrogen atom by way of a C₂-C₈-alkylene or p-xylylene group;
X is a carboxylate, sulfonate, phosphate or phosphonate group;
B is a cosmetically acceptable amine base which may contain siloxane-containing units;
n is 1 to 30; and
m is the valency of the amine B.
A and/or B having a siloxane unit.

3. The hair treatment composition according to claim 1, wherein the salt is formed from isophthalic acid, 5-NaSO₃-isophthalic acid, 1,2,4-benzenetricarboxylic acid or 1,3,5-benzenetricarboxylic acid and 2-amino-2-methylpropanol.

4. The hair treatment composition according to claim 2, further comprising a water-insoluble silicone, especially dimethicone.

5. The hair treatment composition according to claim 4, comprising
a) a siloxane-free hairsetting polymer,
a) a salt of the formula I,
c) a water-insoluble silicone.

6. The hair treatment composition according to claim 2, 4 or 5, wherein the salt is formed from a siloxane-containing acid of the formulae
R¹-NHCO-R²-COOH (IV) 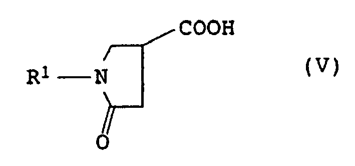
where R¹ is 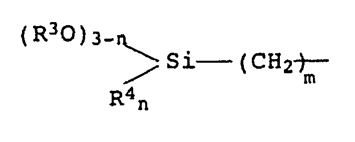 where n = 0-3
and m = 1-6
and R³ and R⁴ are C₁-C₆-alkyl and R² is one of the following radicals:
R² = ―(CH₂)ₘ- where m = 2 - 6
-CH = CH-

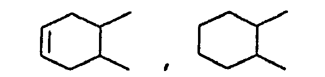

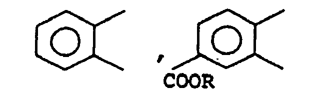

7. The hair treatment composition according to claim 2, wherein the salt is formed from a siloxane-containing monovalent amine of the formula R¹-NR⁹R¹⁰ where R¹ is as defined in claim 6 and R⁹ and R¹⁰, which may be identical or different, are H, C₁-C₆-alkyl or C₁-C₆-hydroxyalkyl.

8. The hair treatment composition according to claim 2, wherein the salt is formed from a siloxane-containing dibasic acid of the formula (VI) or (VII) 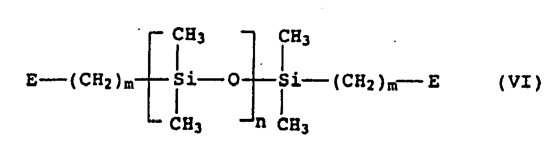 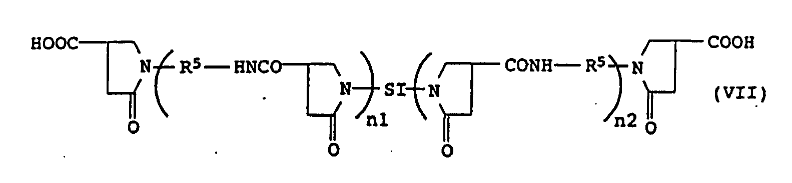 where E is COOH or 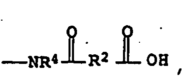 R² is as defined above and R⁴ is H or C₁-C₆-alkyl; m is 1 to 6 and n is 1 to 50, R⁵ is C₂-C₄-alkylene, n1 + n2 = 0-20 and SI is 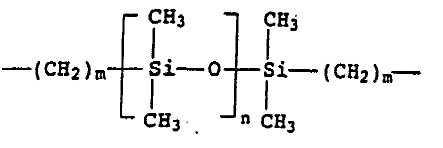 where m is 1 to 6 and n is 1 to 50.

9. The hair treatment composition according to claim 2, wherein the salt is formed from a siloxane-containing divalent amine of the formula (VI) 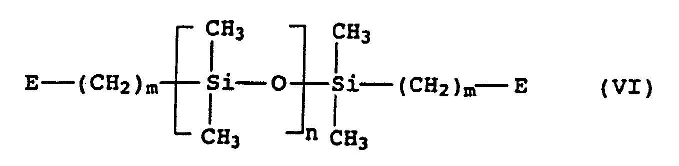 where -E is -NH₂ or NHC₁-C₆-alkyl, m is 1-6 and n is 1-50.

10. The hair treatment composition according to claim 2, wherein the salt is formed from a siloxane-containing polybasic acid of the formula (VIII) 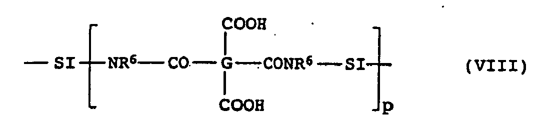 where
R⁶ is H or C₁-C₆-alkyl, SI stands for the definitions indicated in claim 8, p is 1 to 100 and G is the tetravalent radical of a dianhydride 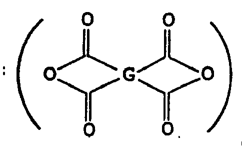 of the formula: 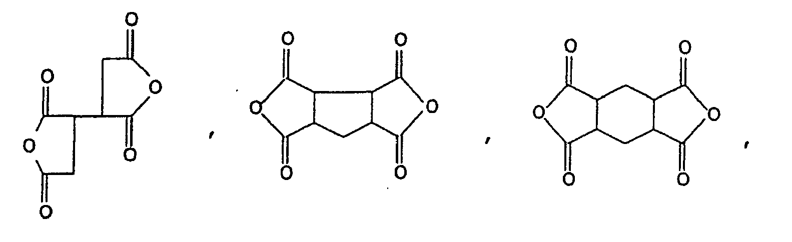 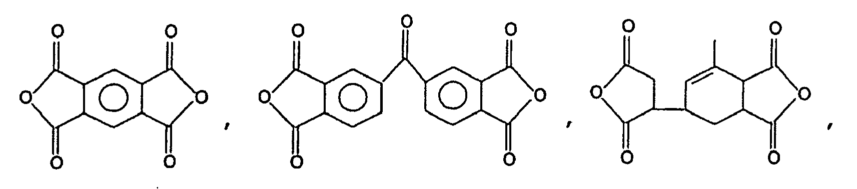 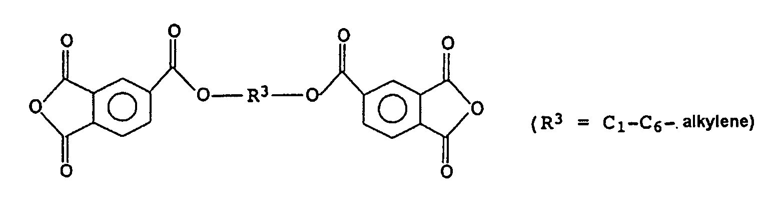 or from polycondensates comprising polysiloxane units of the formula
⁅(̵E―SI―E(̵CONH―R⁷―NHCO⁆ (IX)
where E is O or NH, R⁷ is a C₁-C₆-alkylene or phenylene radical and SI possesses the definitions indicated above, and comprising polyurethane and/or polyurea units of the formula 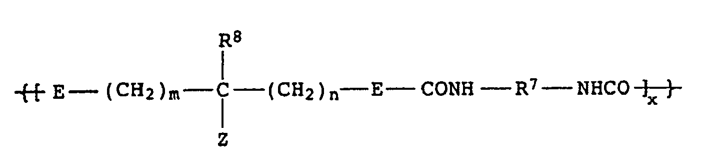 where E is O or NH, R⁷ is C₁-C₆-alkylene or phenylene, R⁸ is H or C₁-C₄-alkyl, Z is COOH or SO₃H, m and n, which may be identical or different, are 1 to 6 and x is 1 to 10.

11. The hair treatment composition according to claim 2, wherein the salt is formed from a siloxane-containing polyvalent amine of the formula VI defined in claim 9 or from polycondensates comprising polysiloxane units of the formula
⁅(̵E―SI―E)̵CONH―R⁷―NHCO⁆ (IX)
where E is O or NH, R⁷ is a C₁-C₆-alkylene or phenylene radical and SI possesses the definitions indicated above, and comprising polyurethane and/or polyurea units of the formula
⁅⁅E― (CH₂)ₘ―L―(CH₂)ₙ―E―CONH―R⁷―NHCO⁆ₓ⁆ (X)
where E is O or NH,
L is 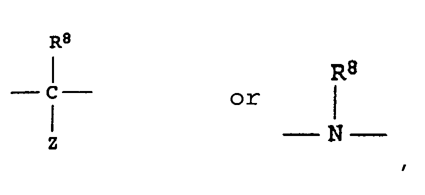 R⁷ is C₁-C₆-alkylene or phenylene, R⁸ is H or C₁-C₄-alkyl, Z is NR¹¹R¹², m and n, which may be identical or different, are 1 to 6, x is 1 to 10 and R¹¹ and R¹², which may be identical or different, are C₁-C₆-alkyl.

12. The hair treatment composition according to claim 4, comprising, based in each case on the overall weight of the composition:
a) from 0.5 to 15% by weight of a siloxane-free hairsetting polymer,
b) from 0.1 to 8% by weight of a salt of the formula I and
c) from 0.0005 to 0.15% by weight, in particular from 0.001 to 0.1% by weight, of silicone.

13. The use of a salt of formula I as defined in one of claims 1 to 11 as an auxiliary in cosmetology or pharmacy, particularly for improving the ease of washing out hairsetting polymers, as a surfactant, emulsifier or protective colloid.

14. The use of the polysiloxane-containing salts defined in one of claims 2 and 4 to 10 as solubilizers for hydrophobic substances, especially silicones, or together with a silicone as auxiliaries in cosmetology or pharmacy.

15. A carboxylic acid of the formula VII defined in claim 8 or salt thereof, especially with a cosmetically acceptable amine base which may contain siloxane- and/or fluorine-containing units.

## Revendications

1. Produit de traitement capillaire, contenant:
a) au moins un polymère fixateur capillaire, soluble ou dispersable dans l'eau ou dans un mélange d'eau et d'un alcool en C₁-C₄ et
b) au moins un sel soluble ou dispersable dans l'eau ou dans un mélange d'eau et d'un alcool en C₁-C₄, de formule I
[A-(X)ₙ]ⁿ⁻ · HB⁺ I
où A désigne un reste aromatique acceptable en cosmétique, qui peut présenter 1, 2 ou 3 substituants, qui peuvent être identiques ou différents et sont choisis parmi les groupes alkyle en C₁-C₆, alcoxy en C₁-C₆, mono- ou polyhydroxyalkyle en C₁-C₆, hydroxy et amino;
X représente un groupe carboxylate;
B désigne une base aminée acceptable en cosmétique;
n vaut de 2 à 3; et
où la base aminée B
est choisie parmi les di-(alkyl en C₁-C₆)-éthanolamines, les mono-(alkyl en C₁-C₆)-diéthanolamines, la monoéthanolamine, la diéthanolamine, la triéthanolamine, le 2-amino-2-méthylpropanol et le 2-amino-2-méthylolpropanol.

2. Produit de traitement capillaire, contenant:
a) au moins un polymère fixateur capillaire, soluble ou dispersable dans l'eau ou dans un mélange d'eau et d'un alcool en C₁-C₄ et
b) au moins un sel soluble ou dispersable dans l'eau ou dans un mélange d'eau et d'un alcool en C₁-C₄, de formule I
[A-(X)ₙ]ⁿ⁻ · [HₘB]^{m+} I
où A désigne un reste aliphatique, cycloaliphatique ou aromatique acceptable en cosmétique, qui peut présenter des unités contenant du siloxane et/ou 1, 2 ou 3 substituants, qui peuvent être identiques ou différents et sont choisis parmi les groupes alkyle en C₁-C₆, alcoxy en C₁-C₆, mono- ou polyhydroxyalkyle en C₁-C₆, hydroxy et amino, tandis que la chaîne du reste aliphatique peut éventuellement être interrompue par jusqu'à 30 groupes -CONH- et que le cycle du reste cycloaliphatique peut présenter un groupe -CO-N-, et tandis que deux restes cycloaliphatiques sont à chaque fois liés à l'atome N par l'intermédiaire d'un groupe alkylène en C2-C8 ou d'un groupe p-xylylène;
X représente un groupe carboxylate, sulfonate, phosphate ou phosponate;
B désigne une base aminée acceptable en cosmétique, qui peut présenter des unités contenant du siloxane;
n vaut de 1 à 30; et
m désigne la valence de l'amine B,
où A et/ou B présente une unité siloxane.

3. Produit de traitement capillaire selon la revendication 1, **caractérisé par le fait que** le sel est formé à partir d'acide isophtalique, d'acide 5-NaSO₃-isophtalique, d'acide 1,2,4-benzènetricarboxylique ou d'acide 1,3,5-benzènetricarboxylique et de 2-amino-2-méthylpropanol.

4. Produit de traitement capillaire selon la revendication 2, **caractérisé par le fait qu'**il contient en outre un silicone insoluble dans l'eau, en particulier de la diméthicone.

5. Produit de traitement capillaire selon la revendication 4, **caractérisé par le fait qu'**il contient
a) un polymère de fixation des cheveux exempt de siloxane,
b) un sel de formule I,
c) un silicone insoluble dans l'eau.

6. Produit de traitement capillaire selon la revendication 2, 4 ou 5, **caractérisé par le fait que** le sel est formé à partir d'un acide contenant du siloxane de formules
R¹-NHCO-R²-COOH (IV) 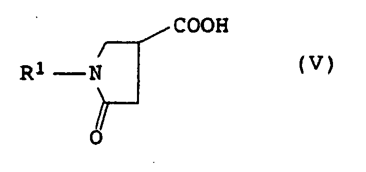
où R¹ désigne 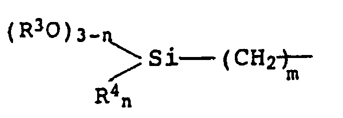 avec n = 0-3
et m = 1-6,
tandis que R³ et R⁴ représentent un alkyle en C₁-C₆ et R² désigne l'un des restes suivants:
R² = ―(CH₂)̵ₘ avec m = 2 - 6
-CH = CH-

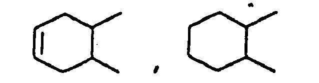

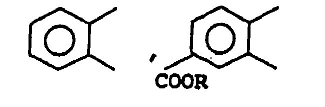

7. Produit de traitement capillaire selon la revendication 2, **caractérisé par le fait que** le sel est formé à partir d'une amine monovalente, contenant du siloxane, de formule R¹-NR⁹R¹⁰, dans laquelle R¹ possède les significations indiquées dans la revendication 6 et R⁹ et R¹⁰, qui peuvent être identiques ou différents, désignent H, un alkyle en C₁-C₆ ou un hydroxyalkyle en C₁-C₆.

8. Produit de traitement capillaire selon la revendication 2, **caractérisé par le fait que** le sel est formé à partir d'un acide divalent, contenant du siloxane, de formule (VI) ou (VII) 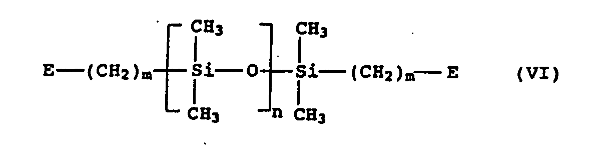 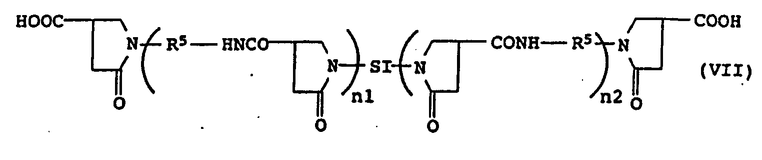 où E représente COOH ou 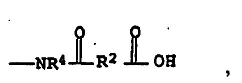 où R² à la signification mentionnée ci-dessus, R⁴ désigne H ou alkyle en C₁-C₆, m vaut de 1 à 6 et n de 1 à 50, R⁵ désigne alkyle en C₂-C₄, n1 + n2 = 0-20 et SI désigne 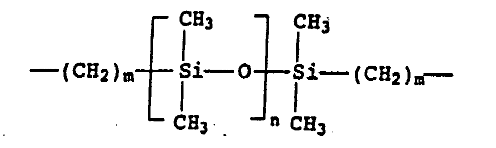 où m vaut 1 à 6 et n vaut de 1 à 50.

9. Produit de traitement capillaire selon la revendication 2, **caractérisé par le fait que** le sel est formé à partir d'une amine divalente, contenant du siloxane, de formule (VI) 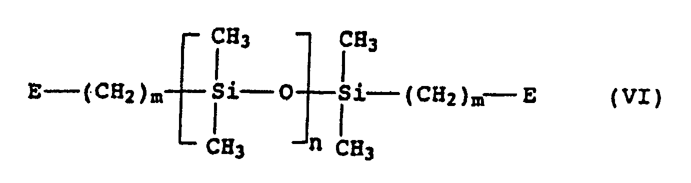 où -E désigne -NH₂ ou un NH-alkyle en C₁-C₆, m vaut 1-6 et n vaut 1-50.

10. Produit de traitement capillaire selon la revendication 2, **caractérisé par le fait que** le sel est formé à partir d'un acide polyvalent, contenant un siloxane de formules (VIII) 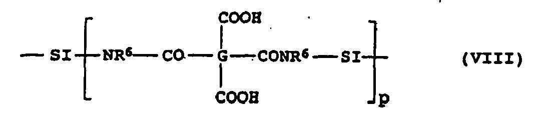 où
R⁶ désigne H ou un alkyle en C₁-C₆, SI possède les significations selon la revendication 8, p vaut de 1 à 100 et G représente le reste tétravalent d'un dianhydride 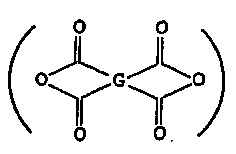 de formule 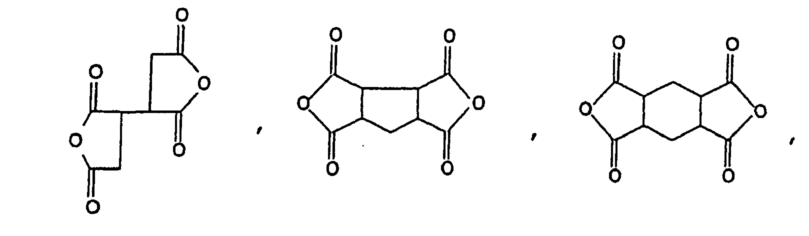 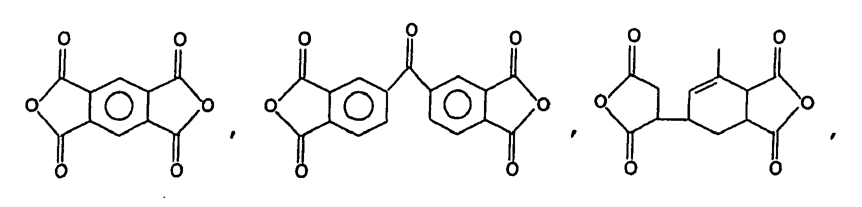 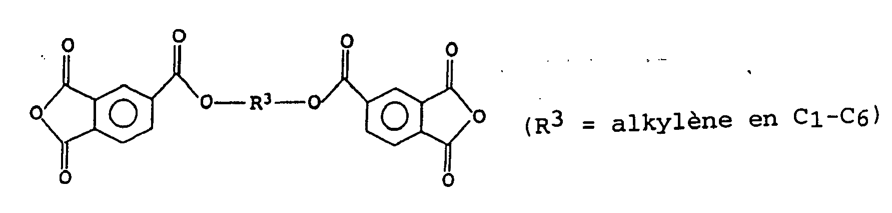 ou à partir de polycondensats avec des unités polysiloxane de formule
[(E―SI―E)―CONH―R⁷―NHCO―] (IX)
où E désigne O ou NH, R⁷ représente un alkylène en C1-C6 ou un reste phénylène et SI possède les significations indiquées plus haut, et avec des unités polyuréthanne et/ou polyurée de formule 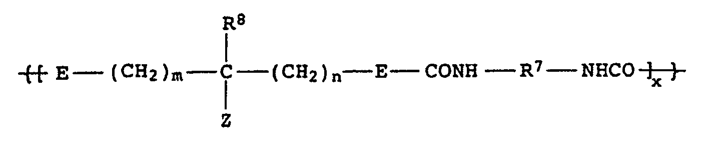 où E désigne O ou NH, R⁷ représente un alkylène en C₁-C₆ ou un phénylène, R⁸ représente H ou un alkyle en C₁-C₄, Z désigne COOH ou SO₃H, m et n, qui peuvent être identiques ou différents, valent de 1 à 6, et x vaut de 1 à 10.

11. Produit de traitement capillaire selon la revendication 2, **caractérisé par le fait que** le sel est formé à partir d'une amine polyvalente, contenant du siloxane, selon la formule VI définie dans la revendication 9 ou à partir de polycondensats avec des unités polysiloxane de formule
[[E―SI―E)-CONH―R⁷―NHCO]- (IX)
où E désigne O ou NH, R⁷ représente un este alkylène en C₁-C₆ ou un reste phénylène, et SI possède les significations indiquées plus haut, et avec des unités polyuréthanne et/ou polyurée de formule
[[E―(CH₂)ₘ―L―CH₂)ₙ―E―CONH―R⁷―NHCO]ₓ] (X)
où E représente O ou NH,
L désigne 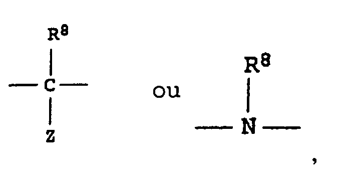 R⁷ représente un alkylène en C₁-C₆ ou un phénylène, R⁸ désigne H ou un alkyle en C₁-C₄, Z représente NR¹¹R¹², m et n, qui peuvent être identiques ou différents, valent de 1 à 6, x vaut de 1 à 10, et R¹¹ et R¹², qui peuvent être identiques ou différents, désignent un alkyle en C₁-C₆.

12. Produit de traitement capillaire selon la revendication 4, **caractérisé par le fait qu'**il contient, à chaque fois par rapport au poids total du produit:
a) 0,5 à 15 % en poids d'un polymère fixateur capillaire exempt de siloxane,
b) 0,1 à 8 % en poids d'un sel de formule I, et
c) 0,0005 à 0,15 % en poids, en particulier 0,001 à 0,1 % en poids de silicone.

13. Utilisation d'un sel de formule I, tel que défini dans l'une des revendications 1 à 11, comme adjuvant en cosmétique ou en pharmacie, en particulier pour l'amélioration de l'aptitude à l'élimination par lavage de polymères fixateurs capillaires, comme agent de surface, émulsifiant ou colloïde protecteur.

14. Utilisation des sels contenant du polysiloxane définis dans les revendications 2 et 4 à 10 comme agents de solubilisation pour des substances hydrophobes, en particulier des silicones, ou conjointement avec du silicone comme adjuvant en cosmétique ou en pharmacie.

15. Acides carboxyliques de formule VII définie dans la revendication 8 ou leurs sels, en particulier avec une base aminée acceptable en cosmétique, qui peut présenter des unités contenant du siloxane et/ou du fluor.
